# EUROPEAN PATENT APPLICATION

(11) **EP 1 133 957 A1**
(43) Date of publication of application: **19.09.2001**
(21) Application number: 00890075.5
(22) Date of filing: 14.03.2000
(51) Int. Cl.: A61F 2/30, A61C 8/00

(54) **Implants with bone ingrowth surface and process therefor**

(71) Applicant: Hiswill Research & Development, Okemos, Missouri 48864 (US)
(72) Inventor: Archbold, Jeff D., Etobicoke, Ontario M9B 1C4 (CA)
(74) Representative: Haffner, Thomas M., Dr.

(57) **Abstract**

A process to produce a bone in-growth surface is described. Bone tissue grows into the in-growth surface of the implanted prostheses to anchor the artificial joint mechanically. The process machines the macroscopic surface of the prosthetic implant by, inter alia, wire-electrodischarge machining, waterjet cutting, laser machining, such that channels (20) at least 50µm in size and up to, but not limited to, 3mm in size are created tangentially to the surface (10). These channels (20) contain undercut regions (24) which provide excellent long-term fixation of the implant in bone tissue. The machining process creates a micro-roughness (30) on the microscopic surface of the channels which enhances early in-growth of bone tissue, reducing healing time. The process to create the bone in-growth surface does not compromise the use of Morse's Taper to improve initial fixation. The in-growth channels (20) formed by the process reduce failure of implants by crack propagation fatique through a diminution of stress concentration regions. The invention is applicable to various configurations of protheses.

## Description

### BACKGROUND OF THE INVENTION

### 1. Field of the Invention

The present invention relates to a process to form a bone tissue in-growth surface for orthopedic and dental implants. The process creates a surface with the attributes of both porous-surfaced implants and microtextured surfaces in one step. The surface created by the process minimizes stress concentrations within prostheses providing for increased fatigue strength over conventional porous surfaces. The invention also relates to articles formed from such process.

### 2. Description of Prior Art

When early prostheses were employed, they were typically implanted using a cement-like agent such as polymethylmethacrylate to bond the implant into a hollowed out portion of the bone of the patient (a more common example is the femoral component in the example of an artificial hip). In such method, it was found that the cement often failed under prolonged and repeated stresses, causing the bond between the implant and the bone to weaken. For this reason, recipients were often encouraged to limit their physical activity. In some cases, a surgeon would not implant the prosthesis if there was a chance that the prosthetic hip might eventually require replacement at an age when the patient would be subjected to undue risks from surgery. The proposed recipient would often have to wait several years before a dysfunctional hip could be replaced.

The next advancement in prosthetic implants used cementless designs. In these cases, the prosthesis was provided with a special coating on the macroscopic surface which permitted the in-growth of bone tissue into the voids/pores of the in-growth surface, forming a mechanical interlock between bone tissue and the implant. As the bone tissue can grow and repair itself, the bond between the implant and the bone is constantly maintained, reducing the possibility of loosening of the implant which was previously a major concern. Different examples of these in-growth surfaces are described in U.S. Patent Nos. 3,605,123, 3,808,606, 3,855,638, 4,636,219, and 4,994,759.

In the prior art processes, the bone in-growth surface is formed using, inter alia, sintered beads, "dovetails", weaves, and capped pillars. These surfaces provide regions into which the bone tissue may grow, but each of these surfaces also create sharp comers, points, and cusps in the implant which are prone to high stress concentrations. These stress concentrations can eventually cause components of the in-growth surface to break from the implant due to fatigue failure. Fatigue failure is caused by the growth of microcracks due to the repeated application of loads. Components of the in-growth surface which break off from the macroscopic surface of the implant can float through the bloodstream contaminating the body and can become lodged in various areas, such as the acetabular cup, causing great wear in the artificial joint. Wear in the artificial joint can become so severe as to necessitate implant replacement. As portions of the in-growth surface break from the implant, the bond between the bone and the implant weakens. To reduce stress concentrations, and therefore to maintain the integrity of the implant, sharp edges and comers must be avoided as much as possible when forming the voids and grooves of an in-growth surface.

To prevent the failure of the implants, the cross-sectional area of the implant can be increased. This reduces the average stress to which the implant is subjected. Increasing the cross-sectional area of the implant makes the implant stiffer and capable of supporting more weight. As the implant supports more weight, the need for bone surrounding the implant is reduced, and the nature of bone remodeling reabsorbs bone tissue from around the implant. This leads to thin bone around large implants which increases the probability of bone fracture making further implant revision increasingly difficult. Another method used to increase the fatigue life of an implant is to avoid placing stress-raising in-growth surfaces in regions of the implant that experience the greatest stresses. Bone tissue will be reabsorbed by the body if it is not stressed, thus the elimination of bone in-growth regions which experience the greatest stress eliminates the regions where bone tissue would best anchor the implant.

Previous art, such as U.S. Patent No. 4,272,855, suggests a means to eliminate comers, cusps, and straight edges but does not provide the three-dimensional mechanical interlock between bone and the implant that is essential to avoid loosening of the implant. Other previous art has used grooves running parallel to the axis of insertion which does not provide a stable long-term fixation system as the shear forces which can separate the bone tissue from the surface of the implant cannot be adequately countered.

Morse's Taper describes the condition where two objects lock into one another when the conical, tapered inner surface of one object matches with the conical, tapered outer surface of the second object. Sintered particles or wire meshes inhibit the close matching of the outer surface of the implant with the inner surface of the bone, limiting the use of Morse's Taper to small, specific regions of an implant to improve the initial fixation of an implant. If there is micromotion of the implant relative to the organic tissues surrounding the implant during the initial healing phase after implantation, fibrous tissue, not bone tissue, will encapsulate the implant. The fibrous tissue compromises implant viability and may require implant revision. An in-growth surface which maximizes the area of the implant available for Morse's Taper anchoring would improve the success rate of the implant surgery.

Some designs use ceramic coatings or plasma-sprayed coatings to promote the fixation of the implant into bone. These coating methods provide an irregular surface for the implant which has been shown to enhance the initial healing and orchestration of bone tissue on the surface of the implant. The roughness of these surfaces, as measured by the peak-to-valley distance, is in the order of 10-20µm. These coatings, however, do not exhibit the three-dimensional interlock of bone and implant which is considered necessary for long-term fixation.

In US patent 4,623,349, an implantable prosthesis is disclosed wherein grooves are provided on the surface of the prosthesis and along the longitudinal axis thereof. The grooves are so oriented to allow for easy removal of the prosthesis after implantation, and as such do not provide superior interlock with bone tissue to improve implant fixation.

In US patent 4,865,608, a prosthesis is disclosed wherein a plurality of grooves are provided for bone adhesion. It is indicated that such grooves are oriented perpendicularly to the direction of force applied to the prosthesis after implantation. This reference does not teach grooves which have a wider undercut region for maintaining three-dimensional mechanical interlock with bone tissue.

US patent 4,608,053 teaches a hip prosthesis wherein the stem portion of the prosthesis is provided with a thickened region including a plurality of ribs on two sides of the prosthesis. Such ribs are provided for mainly to transmit medial loads on the prosthesis to the bone of the femur. It is indicated that such ribs are provided on enlarged sections only of the prosthesis stem so as to prevent the stem from being too rigid.

There is, therefore, a need for a method of producing an article having a bone in-growth surface that minimizes damaging stress concentrations, allows for a mechanical interlock with bone tissue, possesses surface roughness within the in-growth region for accelerated orchestration of bone tissue, and which can utilize Morse's Taper to a significant degree in order to improve the initial fixation of the implant.

### SUMMARY OF THE INVENTION

In one embodiment, the present invention serves to provide a method to produce a minimal stress-concentration bone in-growth surface exhibiting undercut regions with an incorporated microroughness that does not significantly compromise the use of Morse's Taper, and is economically viable.

The in-growth surface is formed by, among others, the firing of a high intensity laser to generate the desired geometric profile, the use of wire electrodischarge machining, the use of waterjet machining, the use of high speed machining, the use of chemical etching, or any combination of these. The process creates tangential channels into the macroscopic surface of the implant into which bone tissue may grow. The channels are shaped such that there are no concave cusps in the implant.

### BRIEF DESCRIPTION OF THE FIGURES

Figure 1 is a cross-sectional diagram of an in-growth channel showing the micro-roughness within the channel.

Figure 2 is a diagram of a femoral component of an artificial hip prosthesis showing machined in-growth channels.

Figure 3 is an enlarged view of the in-growth channels as machined onto a "plate-type" implant.

Figure 4 is an enlarged view of the in-growth channels as machined onto a "conic section type" implant or dental implant.

Figure 5 is a diagram of a dental implant, without the tooth cap, showing the machined in-growth channels.

Figure 6 is a photomicrograph of a sintered powder porous-coated in-growth surface for orthopedic implants.

Figure 7 is a photomicrograph of a plasma-sprayed hydroxylapatite surface for orthopedic implants.

Figure 8 is a photomicrograph of the cross-section of the channel formed by the invention.

Figure 9 is a photomicrograph of the planar view of the channel formed by the invention on a "conic section type" specimen.

Figure 10 is a photomicrograph of the micro-roughness within the channel formed by the invention by wire electrodischarge machining.

Figure 11 is a photomicrograph of the micro-roughness within the channel formed by the invention by waterjet machining.

### DETAILED DESCRIPTION OF THE FIGURES

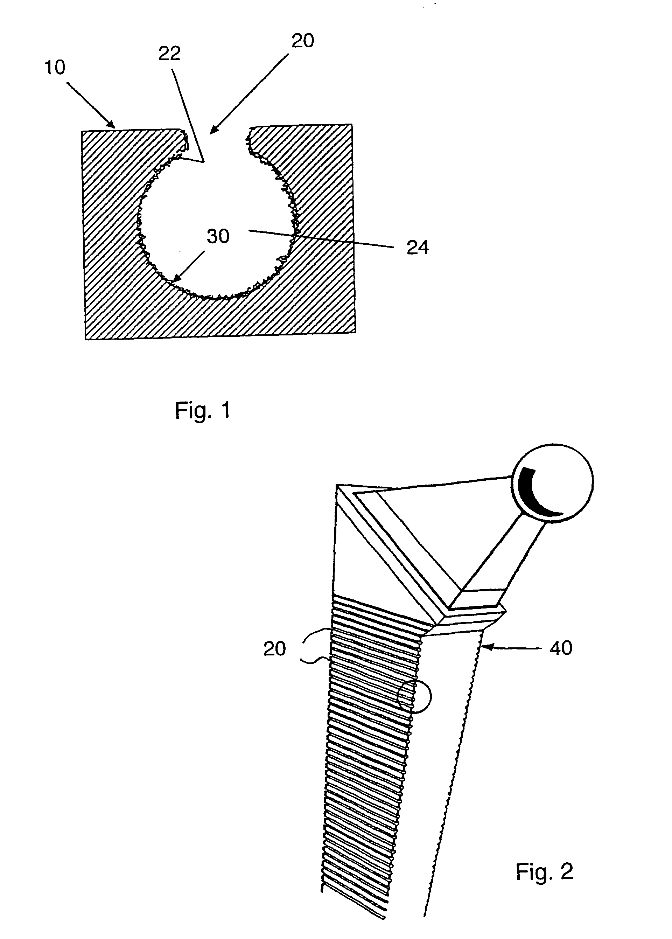

An example of a cross-sectional view of an in-growth channel is shown in Figure 1. The channel 20 is machined tangentially into the macroscopic anchoring surface of the implant 10. The anchoring surface is the surface of the implant or prosthesis that is implanted into bone tissue. By tangentially it is meant that the channels are linear and lie on the surface of the implant. Thus, for a flat implant, such as a plate, the channels would be parallel with the implant surface.

In the preferred embodiment, the opening 22 of the channel should be at least 50µm across, which allows for bone producing and remodeling cells to enter the channel. This ensures that there is the possibility for long term maintenance of the implant/bone interface by the body. It will be appreciated that the any opening of the channel may be possible with the stipulation that such opening must be sufficient to allow in-growth of bone tissue. It has been found that openings of less than 50µm do not permit bone in-growth. Further, although the channel openings can be up to 5000µm, the preferred size would depend upon the type of implant. For example, in orthopedic uses, the channel openings should be approximately 250µm to 500µm. However, for dental implants, openings of 75µm to 150µm are preferred.

The process to machine the channel leaves a machining artifact 30 which enhances the deposition of bone tissue around the implant immediately following surgery. The undercut region 24 of the channel provides a location for bone in-growth and, therefore, a three-dimensional interlock between implant and bone tissue which is beneficial in improving the long term fixation of the implant.

The undercut region is defined as the base of the channel, interior of the implant outer surface. Such a region occurs due to the size of the body of the channel (the undercut region, 24) being larger than that of the channel opening 22. In a preferred embodiment, the undercut region is approximately 50% greater than the size of the channel opening. However, it will be appreciated that any variation in this difference can be used. It will be noted that in instances where the size of the undercut region is the same as that of the channel opening, the result is a groove on the implant surface. The process of the invention allows precise control of the shape of the channel 20 which permits the machining of channels without sharp corners or cusps, which would reduce the fatigue life of an implant.

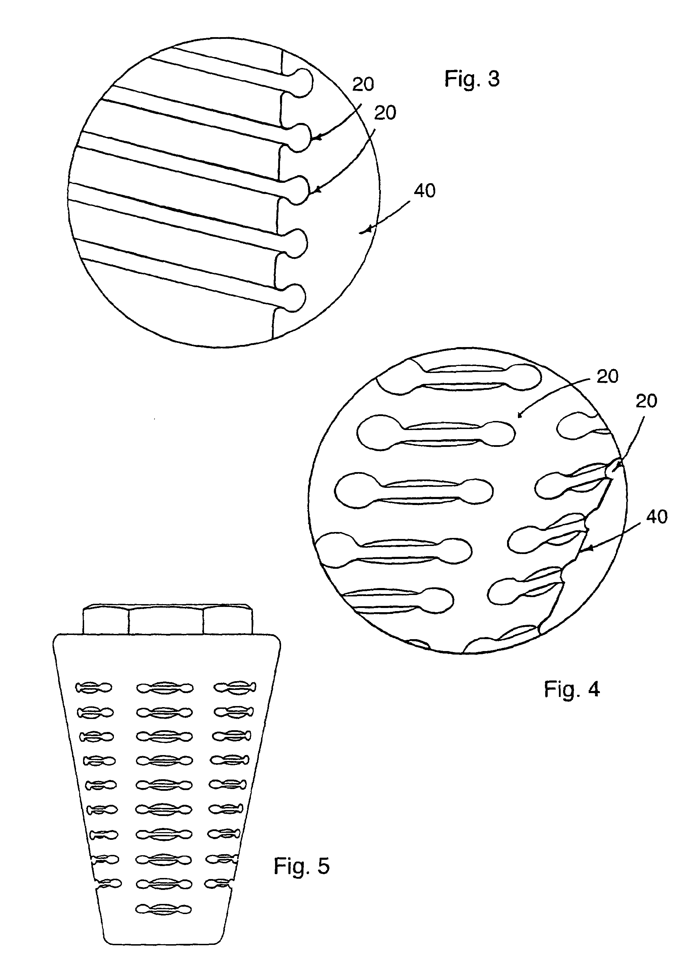

Figure 2 shows a femoral component of a hip implant 40 with a multitude of in-growth channels 20 machined into the surface. The orientation of the channels can be chosen to optimize the fatigue limit of the implant. An enlarged view of the in-growth channels at the edge of a "plate-type" implant is shown in Figure 3. Figure 4 shows an enlarged view of the in-growth channels as machined onto an a "conic-section type" implant. A side view of a dental implant, which is an example of a "conic-section type" implant, is shown in Figure 5. This implant is inserted into the jaw and allowed to heal before the synthetic tooth cap (not shown) is placed over the implant. As shown, the in-growth channels can be oriented perpendicular to the axis of maximum stress since dental implants experience compressive loads almost exclusively. Compressive loads do not promote crack propagation to the same extent as tensile loads. The choice of the machining pattern shown would be more economically viable than to offset the orientation of the in-growth channels from the axis of principle stress for the case of dental implants.

As indicated above, the orientation of the grooves is dependent upon two main criteria, namely, bone adhesion and fatigue stress. In order to achieve maximal anchoring of the implant in bone tissue, the grooves thereon should be parallel to the direction of force applied on the implant. However, fatigue stress on the implant with such groove orientation would lead to the development of micro-cracks in the implant which are then propagated along the grooves. Therefore, to avoid such crack propagation, it would be desirable for the grooves to be perpendicular to the direction of force applied. To achieve a balance between these extremes, the present invention provides for implants having grooves oriented between 0° and 75° to the applied force. For example, as mentioned above, with dental implants, the applied forces are compressive in nature and, therefore, fatigue stress of the implant would not be an issue. For this reason, implants of this type would be designed with grooves which maximize bone anchoring. That is, grooves on dental implants would be oriented at 0°, i.e. parallel, to the applied force. However, with other bone implants (such as hip implants), such parallel orientation is not preferred due to the non-compressive forces which would be applied. In these cases, grooves oriented at between 45° and 75° to the applied force would be desirable.

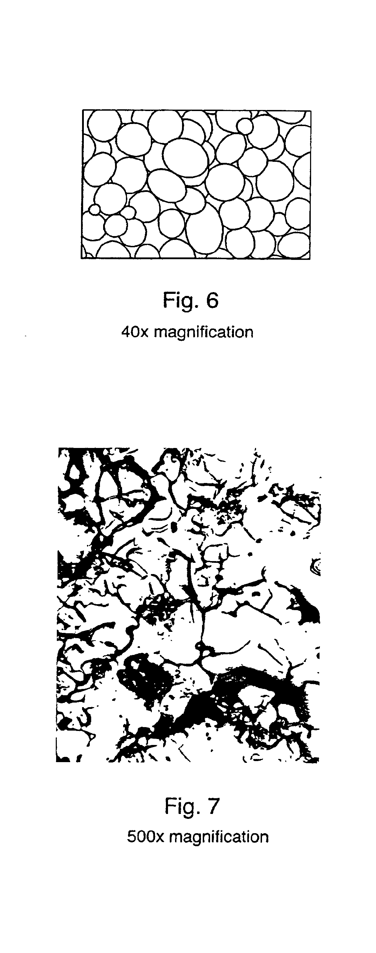

As known in the art, porous-coated implants are chosen for their long term fixation qualities which allow patients to remain more active. A photomicrograph of a sintered powder porous-coating as used on current implants is shown in Figure 6. The spherical powder particles shown are rated at 150µm to 250µm. Bone tissue locks into the three-dimensional interconnected pores between the powder particles to anchor the implant. In the present invention, the undercut regions 24 of the in-growth channels 20, as shown in Figure 1, serve the same purpose as the pores of the sintered powder surface. The sinter-bonding zone between the spherical powder and the macroscopic surface of the implant acts as a notch which reduces fatigue life of an implant. The radius of curvature at a sinterneck, that is, the junction between sinter particle and substrate, is in the order of 5µm. Using the process described, the radius of curvature of an in-growth channel which produces the same size pores as found in Figure 6, is in the order of 100µm. This improves the fatigue life of an implant using in-growth channels as created by the process/invention versus traditional porous-coated implants.

Hydroxylapatite coated implants are chosen for their improved healing versus porous-coated implants. Hydroxylapatite is a ceramic that is similar to bone in composition and has been found to improve the healing of bone tissue around an implant. Much of this improved healing is due to the microroughness that exists when the hydroxylapatite is deposited onto the surface of the implant. Figure 7 shows the topography of the coating as applied to an implant under high magnification. Microroughness of an implant surface is preferred since it accelerates bone adhesion. Such term would relate to a peak to peak distance in the roughness of about 2µm to 20µm, and preferably, 5µm to 10µm.

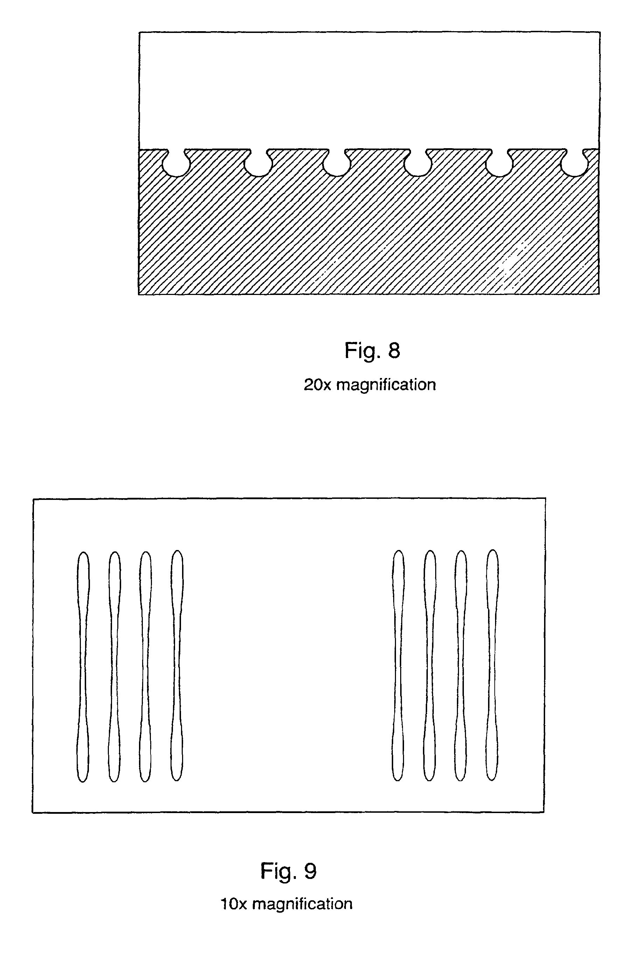

Figure 8 is a photomicrograph of a series of in-growth channels machined into implant grade titanium alloy using wire electrodischarge machining (wire ED). The diameter of the channels is in the order of 400µm. The planar view of a series of in-growth channels machined into a cylindrical specimen of implant grade titanium alloy is shown in Figure 9. It is noted that the macroscopic surface of the specimen is quite smooth where it is not machined. This will allow for the significant use of Morse's Taper to improve initial fixation of an implant when the in-growth surface is created by the process of the invention.

The channels illustrated in Figures 8 and 9 were formed using a 200µm (0.008") diameter wire and into a Ti-6Al-4V alloy. This alloy is a standard for orthopedic implants.

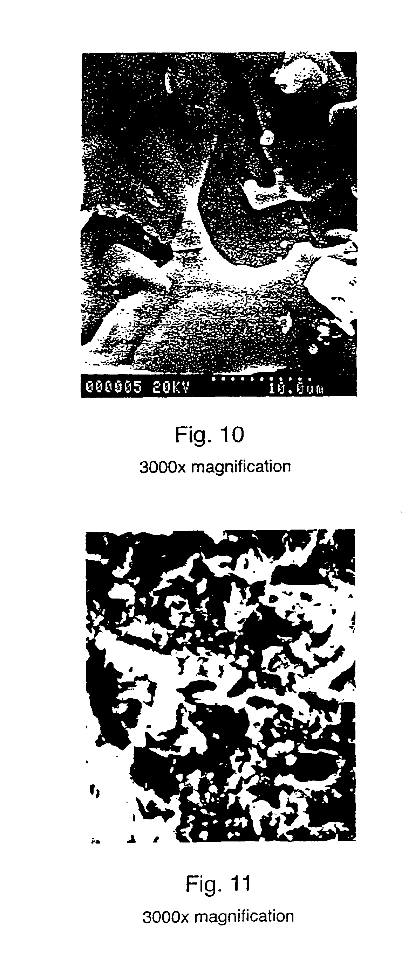

An enlarged view of the microroughness as produced by wire electrodischarge machining is shown in Figure 10. Note the similarity to the plasma-sprayed coating of Figure 7. The channels shown in Figure 10 were formed with a 75µm (0.003") wire and into a Ti-6Al-4V alloy. This diameter of wire would be preferred for dental implants due to the small size of such articles. However, the topography of the implant would be the same for all diameter wires.

The microroughness of an in-growth channel formed by waterjet machining is shown in an enlarged view in Figure 11. The channels shown in Figure 11 were formed in a Ti-6Al-4V alloy with a pure water (no grit) waterjet emitted by a 200µm (0.008") nozzle.

According to a preferred embodiment of the invention, the in-growth channels are repeated parallel, or roughly parallel, to one another over the region of the prosthesis where the in-growth surface is desired. For applications where the greatest possible fatigue strength is required, the channels are oriented such that the plane of principle stress does not run parallel with the direction of the channels. The channels must still be oriented such that they do not run parallel with the axis of insertion, as this would reduce the ability of the in-growth surface to counter shear stresses imposed though axial loading of the implant. In the case of a dental implant, the channels can be oriented perpendicular to the insertion axis as this provides the optimum geometry to resist shear stresses and the dental implant is almost always stressed in compression. The problem of small crack growth under cyclic fatigue is reduced when there is no tensile force to promote crack opening.

For a "plate type" implant, that is, an implant that is based on a flat plate for the portion of the implant that is inserted into the bone, the channels are formed on each side of the plate. The channels are oriented such that they run neither parallel to the long axis of the implant (the axis along which the implant is inserted into the bone), which would not adequately resist shear forces between the implant and the femur, nor perpendicular to the greatest tensile forces experienced by the implant, which would reduce fatigue life.

For a "conic section type" implant, that is an implant in which the portion of the implant that is inserted into the bone has a circular, oval, elliptical, etc. cross-section, the channel is formed tangentially to the curved macroscopic surface of the implant. This process is repeated, with subsequent channels not overlapping the previous channels. In this manner a ring of channels about the macroscopic surface of the implant is generated over the region of the implant where the in-growth surface is desired. The orientation of the channels is again chosen to resist shear and minimize fatigue strength reduction due to tensile forces.

The density of the channels on the implant surface is related to the fatigue failure characteristics of the implant. Specifically, a higher density of channels results in a decrease in the stress cracks which can form on the implant. These stress cracks are subcritical cracks in the order of 100µm in width. In the preferred embodiment, the separation between adjacent undercut regions of the channels is about 200µm to 250µm. It will be appreciated that the separation between adjacent channels would depend upon the diameter of the undercut region.

It will be apparent to persons skilled in the art that various methods are possible for creating the channels as above in the implant and various suggested methods are discussed above. These methods include, but are not limited to, wire electrodischarge machining, laser machining, waterjet machining, and chemical etching. Of these methods, wire electrodischarge machining would be preferred since it provides the most consistent results and allows for smaller channel sizes.

Although the invention has been described with reference to certain specific embodiments, various modifications thereof will be apparent to those skilled in the art without departing from the spirit and scope of the invention as outlined in the claims appended hereto.

## Claims

1. A process for forming channels on the outer surface of an implantable prosthesis, said channels being adapted for bone tissue in-growth and having an opening on said prosthesis surface opening into an undercut region interior of said surface.

2. The process of claim 1 wherein said undercut region has a width greater than that of said opening.

3. The process of claim 2 wherein said channels are formed using methods including: wire electrodischarge machining, laser machining, waterjet machining, and chemical etching.

4. The process of claim 3 wherein said channels are tangential to said prosthesis surface.

5. The process of claim 4 wherein said channels include a microroughness sufficient to promote bone tissue in-growth.

6. The process of claim 5 wherein said microroughness is 5µm to 20µm.

7. The process of claim 6 wherein said channel openings are at least 50µm

8. The process of claim 7 wherein the width of said undercut region is about 50% greater than the width of said opening.

9. The process of claim 1 wherein said channels are oriented between 0° and 75° to the direction of the force applied on said prosthesis after implantation.

10. The process of claim 1 wherein said prosthesis is a dental prosthesis and said channels are parallel to the force applied on said prosthesis after implantation.

11. The process of claim 1 wherein said channels are oriented between 45° and 75° to the force applied on said prosthesis after implantation.

12. An implantable prosthesis having an anchoring surface for implanting into bone tissue, said anchoring surface having a plurality of channels having an opening on said anchoring surface and an undercut region interior of said anchoring surface, said channels being adapted for permitting bone growth therewithin.

13. The prosthesis of claim 12 wherein said undercut region has a width greater than that of said opening.

14. The prosthesis of claim 13 wherein said channels are tangential to said anchoring surface.

15. The prosthesis of claim 14 wherein said channels include a microroughness sufficient to promote bone tissue in-growth.

16. The prosthesis of claim 15 wherein said microroughness is 5µm to 20µm.

17. The prosthesis of claim 16 wherein said channel openings are at least 50µm

18. The prosthesis of claim 17 wherein the width of said undercut region is about 50% greater than the width of said opening.

19. The prosthesis of claim 12 wherein said channels are oriented between 0° and 75° to the direction of the force applied on said prosthesis after implantation.

20. The prosthesis of claim 12 wherein said prosthesis is a dental prosthesis and said channels are parallel to the force applied on said prosthesis after implantation.

21. The prosthesis of claim 12 wherein said channels are oriented between 45° and 75° to the force applied on said prosthesis after implantation.
